# EUROPEAN PATENT APPLICATION

(11) **EP 4 714 458 A2**
(43) Date of publication of application: **25.03.2026**
(21) Application number: 25213329.3
(22) Date of filing: 09.04.2020
(51) Int. Cl.: A61K 35/00

(54) **CONTINUOUS TREATMENT WITH PLASMA**

(30) Priority: 10.04.2019 EP 19168499
(62) Divisional of application: 20715938.5
(71) Applicant: NexaBiome Limited, Glasgow G20 0SP (GB)
(72) Inventor: CLARK, Jason Richard, Glasgow G20 0SP (GB); MATTEY, Michael, Glasgow G20 0SP (GB)
(74) Representative: Schlich

(57) **Abstract**

The present invention relates to an electrode assembly for exposing an object supported on a moving surface to an electrical discharge comprising: (a) an electrical discharge device capable of generating an electrical discharge, further comprising an electrode having an electrical discharge generating surface capable of generating an electrical discharge originating from an active portion of the surface, wherein the surface is movable in order to allow generation of an electrical discharge from a different portion of the surface; (b) a barrier located in closely spaced separation to an inactive area of the electrode wherein the barrier separates the active portion of the electrode from an inactive portion of the electrode; and (c) means for cooling the inactive portion of the electrode. Methods and uses of the electrode assembly of the invention are also described, in particular exposing an object to an electrical discharge generated by the electrode assembly and further particularly attaching a molecule or macromolecule to an object following exposure of the object to the electrical discharge.

## Description

### Field of the Invention

The present invention relates to methods and apparatus for producing products with molecules or macromolecules attached thereto. Such macromolecules include bacteriophage. Thus products of methods of the invention are for prevention and amelioration of bacterial contamination of the products or methods of the invention or materials in contact with said products.

### Background to the Invention

A bacteriophage ('phage') is a virus that infects and replicates within a bacterium. 'Lytic' bacteriophages cause bacterial cells to be broken open (lysed) and destroyed after replication of the virion within the bacterium. When the cell is destroyed, the phage progeny can find new hosts to infect. Bacteriophages are the most numerous form of life on Earth. They can be found in all environments where bacteria grow. For example, bacteriophages are detected in ground and surface water, soil, food (e.g. sauerkraut, wine), sewage and sludge. They have also been isolated from humans and animals, for example from faeces, urine, saliva, spit, rumen and serum. It is known to apply free bacteriophage to products, for example by spraying. However, such treatments have not been found to be reliably effective in preventing or treating bacterial contamination.

Bacteriophages are able to penetrate different organs and tissues, including the central nervous system, and are a part of intestinal flora together with their bacterial hosts. They are responsible for 10-80% of total bacterial mortality in aquatic ecosystems and are an important factor limiting bacterial populations.

Bacteriophages are able to disperse in both solid and liquid media. International patent application WO 03/093462 discloses that immobilised bacteriophages can be covalently attached to a substrate using chemical methods or an electrical discharge, making it impossible for them to diffuse freely, but retaining their ability to infect cells, thus subsequently causing bacterial lysis and release of free bacteriophage.

In WO 2007/072049 those methods were developed to the use of a pulsed field corona discharge; particles activated by the discharge were dropped into bacteriophage solutions. Similarly, in WO 2012/175749 bacteriophage solutions were combined with activated seeds.

Therapeutic applications of bacteriophage are known. WO 03/093462 discloses methods for the immobilisation of viruses, in particular bacteriophages, whilst retaining their biological activity for use as antibacterial agents. Given that the natural environment of bacteriophages is aqueous it has been widely assumed that stability towards dehydration as disclosed in WO 03/093462 tends towards the natural stability in aqueous media.

WO2012/175749 describes small particles with bacteriophage attached thereto that are suitable for combining with foodstuffs to ameliorate bacterial contamination thereof as disclosed in.

WO2014/049008 describes small particles with bacteriophage attached thereto that are particularly suitable for treating or preventing bacterial infections.

Wang et al (2015) [Reference [1] at the end of the description] have shown that low pressure plasma treatment, a form of electrical discharge treatment, for 1 minute in an oxygen atmosphere enhanced the adhesion of bacteriophages to surfaces.

Such treatment of surfaces, to allow the stable binding of molecules or macromolecules (including phage), is referred to as "activation". Surface activation by corona discharge or other plasma generating electrical methods is well known (see e.g. Barton et al [2]; Canavan et al [3]; Recek et al [4]; Kerkeni et al [5] and others). It is well known in industrial practice, in for example printing, that such electrical treatments result in increased hydrophilicity of surfaces (wettability) and hence improved adhesion. Such increased adhesion due to greater hydrogen bonding differs from the use of rapid contact between the electrically activated surfaces and bacteriophages described in the above patents to achieve covalent bonding.

In addition, US 7,250,195 discloses generating a corona discharge which is introduced into a vacuum chamber for deposition of biomaterial (including amino acids and proteins, but not bacteriophage) onto a substrate.

WO 2014/134297 discloses methods of covalently attaching bacteriophage to surfaces by forming carboxylic acid residues on the surface of the substrate to which the bacteriophage can covalently bond.

In the art, only batch manufacturing methods are practical. For example, materials are placed in a shallow activation vessel and subjected to corona activation from above and then quickly brought into contact with a solution containing the bacteriophages to be immobilised. This method is more or less acceptable for sheet (planar) material with immobilisation occurring at one surface, although it is also used inefficiently for filamentous and particulate material.

Methods for the continuous treatment of articles with corona discharge are known. For example, US 2015/0373923 discloses a plasma treatment device for treating crops, such as sprout plants, to reduce bacterial viability and US 2004/0086433 discloses apparatus comprising a conveyor for exposing an object to a corona discharge. In addition, US 3,017,339 discloses a method and apparatus for treating thermoplastic tubing with a corona discharge. However, methods have not been developed for the continuous treatment of articles with corona discharge in order to attached macromolecules (such as bacteriophage) to articles.

Hence, there is a need for continuous manufacturing methods for producing products with bacteriophage covalently attached thereto. This is particularly the case for products comprising small particles with bacteriophage attached thereto. Methods commonly used in industry, for example, standard corona discharge treatments, are not suitable for the activation of small particles. Unlike films, small particles are subject to dispersion by forces caused by build-up of static electricity; furthermore, blown air is typically used to cool electrodes in these industrial processes, which does not affect films but which can displace small particles, preventing their activation.

### Object of the Invention

An object of the present invention is to provide continuous methods for producing products for the prevention and amelioration of bacterial contamination, and apparatus for manufacturing such products.

### Summary of the Invention

Accordingly, the invention provides a method for attaching a molecule or macromolecule to an object comprising the steps of:
placing the object on or in the proximity of a surface;
controlling the electrical potential of the surface with respect to its surroundings;
activating the object by exposing it to an electrical discharge;
contacting the object with the molecule or macromolecule to be attached.

Objects need not be in direct contact with the surface but in proximity to the surface such that the electrical potential of the surface directly affects and/or defines the electrical potential of the objects.

The electrical potential of the surface, and hence objects in contact with or in proximity to the surface, may be controlled by electrically earthing the surface. Accordingly, the surface may be earthed (grounded). Alternatively, the electrical potential of the surface, and hence objects in contact or in proximity the surface, may be controlled by limiting the charge accumulated by the surface and/or objects in contact or associated with the surface. This may be done by dissipating the charge continuously or by dissipating the charge when the net charge reaches a defined threshold. That is all or part of the excess negative or positive charge accumulated by the surface can be dissipated by grounding or other means. The charge dissipated may have an electrical potential of up to 10,000 volts per centimetre and may produce an energy of 500 millijoules during discharging.

Control of the electrical potential of the surface may conveniently be achieved by making the surface conducting. Accordingly, in order to accommodate grounding the surface or the object that the surface is mounted on or part of it may be conducting. Preferably the surface or the object that the surface is mounted on or part of is partially or entirely metallic and thus intrinsically conducting.

The electrical discharge may be a corona discharge. Generation of corona discharge means that a source of electrical resistance is required within the apparatus containing the corona source, this can be from the electrode, around the grounding/treatment area or both.

The corona discharge generating electrode may be ceramic. Ceramic electrodes are advantageous and particularly suited to the production of corona discharge when the treatment platform is conducting, i.e. comprises metallic elements or conducting materials. In addition, ceramic electrodes are resistant to degradation due to high voltage and thus have an advantageously extended working lifetime.

Use of a ceramic as a resistance around the electrode advantageously distributes the power of the discharge evenly while still yielding an effective and reliable treatment.

Alternatively, a metal electrode with a source of electrical resistance attached to the grounded area may be used in applications when the treatment effect is to jump a greater distance.

In addition, a small electrical resistance is provided by using a conveyor belt comprised in the apparatus or used in the method as this resistance advantageously assists in keeping the effect of the treatment even.

Alternatively or in addition, the electrical potential of the surface may be controlled by a second electrode located in a position whereby it can be used to oppose or modulate the electrical potential caused by exposure of the surface and object borne there on to an electrical discharge.

Preferably, this additional electrode is located on the opposite side of the object and/or surface from the source of the electrical discharge.

In this way the electrical potential of the object on or in contact with the surface is also controlled by the means of controlling the electrical (electrostatic) potential of the surface. This may also be the case for objects in indirect contact with the surface whose electrical potential may also be controlled in concert with the electrical potential of the surface.

The surface is typically a moving surface and thereby allows continuous attachment of molecules/macromolecules to the object(s). The surface may be a conveyor platform. That is a moving surface suitable for carrying objects along a predetermined route. Preferably the surface is a conveyor belt. Accordingly, the second additional electrode for controlling the electrostatic potential of the surface and the objects thereon may be located beneath the conveyor platform.

Hence the invention is particularly suited to continuous treatment processes. A continuous treatment process contrasts with a batch treatment process. In a batch treatment process a set quantity of objects or input substrate is processed by a method with a defined start and end point. In contrast, in a continuous process the amount of material or object processed is defined by the amount of material available and the time that the process is run for. Once the treatment process and the input and output flows are stable then products of the method can be produced for an indeterminate period until the continuous process is stopped.

Necessarily, electrostatic effects are less important or deleterious to batch processes as the opportunity for static charge accumulation is significantly less. Additionally, a batch process can be built around a solid (i.e. non-flexible) surface, such as a metal plate, whereas continuous, belt-driven processes require a flexible surface. Furthermore, the opportunities for natural dissipation of accumulated charge are significantly greater because electrical activation is only used intermittently.

Hitherto, treatment of objects using electrical discharges has been carried out on a wide variety of substrate materials and objects without deleterious effects on the positioning of objects because of an excess of electrical charge. In general, the size and/or mass of the treated object has been sufficient to render trivial any forces due to the presence or build-up of electrical charges.

It was thus unexpected that treatment of particles using electrical discharge would be substantially prevented by them becoming electrically charged during the treatment process. The movement of all or a part of the population of particles being treated means that treatment is rendered less reliable and thus gives treatment to a lesser extent than that expected and/or required.

Accordingly, the present inventors have developed methods and apparatus of the invention that allow control of the electrical potential yielding the advantages of eliminating, reducing or otherwise controlling the electrostatic effects that may act on the object being treated. Small objects are disproportionately affected by static effects in that a static charge can cause small particles to be repulsed or attracted to one another leading to uncontrolled dispersion or uncontrolled clumping. In either case, efficient treatment of the particles is rendered difficult or impossible. Particles which are too highly charged will be repulsed by the electrical discharge, to the extent that they will not pass through the corona and will not be activated. Additionally, a build-up of static electricity can cause small particles to be dispersed in a random and unpredictable manner so that they cannot be efficiently activated by the electrical discharge. Such effects are particularly pronounced, and thus disadvantageous, when the particles are formed into a monolayer.

Furthermore, these electrostatic effects on the object being treated can be caused or exacerbated by their being exposed to an external electrical discharge.

The object provides a substrate for the attachment of the molecule or macromolecule thereto. Accordingly, the terms object and substrate are used interchangeably herein.

The term "substrate" is understood to mean any solid phase material to which a molecule or macromolecule may be immobilised following exposure of the substrate to a suitable electrical discharge. Said substrate may take many forms, for example, nylon and any other polymer with amino or carboxyl surface groups, cellulose or other hydroxyl-containing polymer, polystyrene or other similar polymer, various plastics or microbeads including magnetic particles, biological substances. Said substrate may also be made of a material commonly used in therapy/medicine. For example, nylon thread for use in surgery; plastics, lint or gauze material used to dress open wounds; microbeads, which can be ingested; adhesives such as cyanoacrylates; and/or biological substances such as collagen or hyaluronic acid. More preferably, said material may be edible food or feed particles, containing proteins and bulking material, such as cellulose or other plant-derived polymers.

The term "activated/activating/activation" according to the present invention is understood to mean the activation of a substrate by reacting said substrate with various chemical groups (leaving a surface chemistry able to bind bacteriophage head or capsid groups). Activation of said substrate is achieved by electrical discharge, preferably corona discharge. However other forms of electrical discharge may be used. In particular activation of said substrate may be achieved by applying a pulsed corona discharge and/or an electron beam. Contact between a molecule or macromolecule and the activated substrate leads to the formation of a covalent bond between the molecule or macromolecule and the substrate. Accordingly, use of the term "attaching" in the context of the present invention means covalent attachment.

The substrate may be a particle. The particles may be of a size less than or equal to 2 mm in diameter, preferably less than or equal to 1 mm in diameter, most preferably in the range of 1-1000 µm. The particle may be substantially spherical.

Suitably, the particles may be food pellets. The food pellets are suitable for consumption by humans and/or animals. Preferably the food pellets are suitable for use as animal feed. The pellets may preferably be in the range of 1 mm - 20 mm in height as measured from a surface they are lying or supported on. The pellets may be substantially spherical, cylindrical or spherocylindrical.

In addition, the object may be a seed e.g. a plant seed. Seeds may be advantageously treated according to the invention because of the effect of static electricity on handling small objects such as plant seeds. Seeds suitable for treating according to the invention may be of a size less than or equal to 10 mm in diameter, preferably in the range of 0.2 to 5 mm.

The molecule or macromolecule may be a biomolecule or a biological entity. The biomolecule may be an antibody, cytokine or other signalling molecule. Alternatively, or in addition the molecule or macromolecule may be a biological entity such as multimeric protein or antibody, or a virus. Preferably the macromolecule is a virus and most preferably the macromolecule is a bacteriophage.

The term "bacteriophage" according to the present invention is indicative of bacteriophage that infect specific strains of bacteria. It will be understood to the skilled person that bacteriophage can recognise and infect specific strains of bacteria. Thus, a substrate may be a material which may be advantageously activated to allow head-group specific binding of a complex bacteriophage. Bacteriophage are immobilised via covalent bonds formed between the bacteriophage coat protein and the substrate. More preferably, bacteriophage are immobilised to the substrate via their head groups or nucleocapsid by activating the substrate before the addition and coupling of bacteriophage.

Thus, bacteriophage immobilised to a substrate according to the present invention, may be utilised to fight strain-specific bacterial infections as a "bactericide" by inducing selective killing of bacteria through cell lysis or as a "bacteriostatic agent" by inhibiting bacterial growth. Bacteriophage immobilised to a substrate may also be used as an antibacterial agent/disinfectant in order to "sterilise" bacterially-contaminated material. Accordingly, preferably the bacteriophage retains infectivity when immobilised.

The surface may also comprise cavities or indentations for accommodating particles. The mouths of the cavities or indentations preferably face towards the source of the electrical discharge when the particle therein is activated by the electrical discharge. Preferably, the indentations or cavities are sized to accommodate a single particle.

Preferably the particles accommodated by the cavities or indentions are of 1 cm or greater in diameter. The power required to treat larger particles is significantly higher than for smaller particles. This is because the power requirement follows an inverse-square law versus a linear increase in dimension.

Thus, the surface may be made with indentations into which the particles fit. A proportion of the particle would protrude above the surface of the surface out of the cavity or indentation. Accordingly, the linear range that the corona discharge would have to cover in order to effectively treat the protruding part of the particle would be significantly less. In this way it is possible to activate a significant and effective portion of the particle without having to treat the whole particle. Consequently, a lower power electrical discharge source can be used. Such a lower power electrical discharge source is advantageously less expensive to obtain, run and maintain.

The invention also provides an apparatus for treating an object by attaching a molecule or macromolecule thereto, comprising:
a surface for supporting the particle being treated
means for controlling the electrical potential of the surface with respect to its surroundings;
an electrical discharge device capable of generating an electrical discharge.

The invention also provides for use of the apparatus of the invention for attaching a molecule or macromolecule to particles.

The invention also provides an electrode assembly comprising:
an electrical discharge device capable of generating an electrical discharge, further comprising an electrode having an electrical discharge generating surface capable of generating an electrical discharge originating from an active portion of the surface, wherein the surface is movable in order to allow generation of an electrical discharge from a different portion of the surface.

The electrical discharge may be generated continuously while the electrical discharge generating surface moves

The advantage of the surface of the electrode being movable is that the heat energy that must be dissipated during the operation of the electrode is generated on a specific portion of the electrode that may be changed during use in order to more evenly distribute the heat energy produced during operation. In this way, hotspots on the electrode are avoided and an increased operational lifetime of the electrode is obtained along with increased reliability.

The movable electrode may be rotatable, preferably the electrode is cylindrical. Such a configuration is convenient in that only one direction of movement is required to yield the advantages of moving the electrode during continuous use.

The electrode assembly may further comprise means for cooling the inactive portion of the electrode. Preferably the cooling means is air cooling, most preferably the cooling means is forced air cooling.

The electrode assembly may comprise a barrier located in closely spaced separation to an inactive area of the electrode wherein the barrier separates the active portion of the electrode from an inactive portion of the electrode. This configuration has the advantage of separating "active" side of the electrode in which the object is being activated, from the "inactive" side of the electrode which may be being cooled. In this way the two temperature environments are separated and the cooling mechanism is prevented from affecting the treatment mechanism on the other side of the barrier.

The barrier may be made of rubber or plastic, preferably the barrier is made of silicone material.

The barrier may be in contact with the surface of the electrode or in closely spaced separation from the electrode surface, preferably located 10 to 1000 µm from the electrode surface.

Such materials and configuration have the advantage of allowing the barrier to act as a scraper in order to remove particles or other matter that have adhered to the surface of the electrode. In this way the electrode can be run continuously for more extended periods of time.

The invention also provides for use of the electrode assembly of the invention for attaching a molecule or macromolecule to a substrate.

Features disclosed herein are further described by way of the following numbered paragraphs of embodiments:
1. A continuous method for attaching bacteriophage to an object comprising the steps of:
   (a) placing the object on or in the proximity of a moving surface;
   (b) controlling the electrical potential of the surface with respect to its surroundings;
   (c) activating the object by exposing it to an electrical discharge; and
   (d) contacting the object with the bacteriophage to be attached.
2. A method for attaching bacteriophage to an object according to embodiment 1, wherein the moving surface is conducting.
3. A method for attaching bacteriophage to an object according to embodiment 1 or embodiment 2, wherein the electrical potential of the moving surface is controlled by grounding the platform.
4. A method for attaching bacteriophage to an object according to embodiment 1, wherein the electrical potential of the surface is controlled by generation of an electrostatic potential from an electrode.
5. A method for attaching bacteriophage to an object according to any preceding embodiment, wherein the moving surface is a conveyor belt.
6. A method for attaching bacteriophage to an object according to any preceding embodiment, wherein the electrical discharge is a corona discharge.
7. A method for attaching bacteriophage to an object according to any preceding embodiment, wherein the object is a particle of less than or equal to 2 mm in diameter.
8. A method for attaching bacteriophage to an object according to any preceding embodiment, wherein the object is a particle of less than 1 mm in diameter.
9. A method for attaching bacteriophage to an object according to any preceding embodiment, wherein the particle is a food or feed pellet.
10.A method for attaching bacteriophage to an object according to any preceding embodiment, wherein the surface comprises indentations for accommodating objects.
11.An apparatus for treating an object by attaching bacteriophage thereto comprising:
   (a) a surface for supporting the object being treated;
   (b) means for controlling the electrical potential of the surface with respect to its surroundings;
   (c) an electrical discharge device capable of generating an electrical discharge.
12.An apparatus according to embodiment 11, wherein the surface is a moving surface.
13.An apparatus according to embodiment 11 or 12, for carrying out the method of any of embodiments 1 to 10.
14.Use of the apparatus of any of embodiments 11 to 13 for attaching bacteriophage to an object.
15.An electrode assembly comprising:
   an electrical discharge device capable of generating an electrical discharge, further comprising an electrode having an electrical discharge generating surface capable of generating an electrical discharge originating from an active portion of the surface, wherein the surface is movable in order to allow generation of an electrical discharge from a different portion of the surface.
16.An electrode assembly according to embodiment 15, wherein the electrical discharge is generated continuously while the electrical discharge generating surface moves.
17.An electrode assembly according to embodiment 16, wherein the electrode assembly comprises a barrier located in closely spaced separation to an inactive area of the electrode wherein the barrier separates the active portion of the electrode from an inactive portion of the electrode.

### Examples and Description of the Drawings

The invention is now illustrated in the following specific embodiments with reference to the accompanying drawing:
Fig. 1 shows a schematic diagram of an apparatus 100 as described herein in the process of treating fine particles to attach infectious bacteriophage thereto, as is also described herein.
Fig. 2 shows an isometric diagram of an apparatus 200 as described herein for treating fine particles to attach infectious bacteriophage thereto, as is also described herein.
Fig. 3 shows a cross sectional diagram of an apparatus 200 as described herein for treating fine particles to attach infectious bacteriophage thereto, as is also described herein.

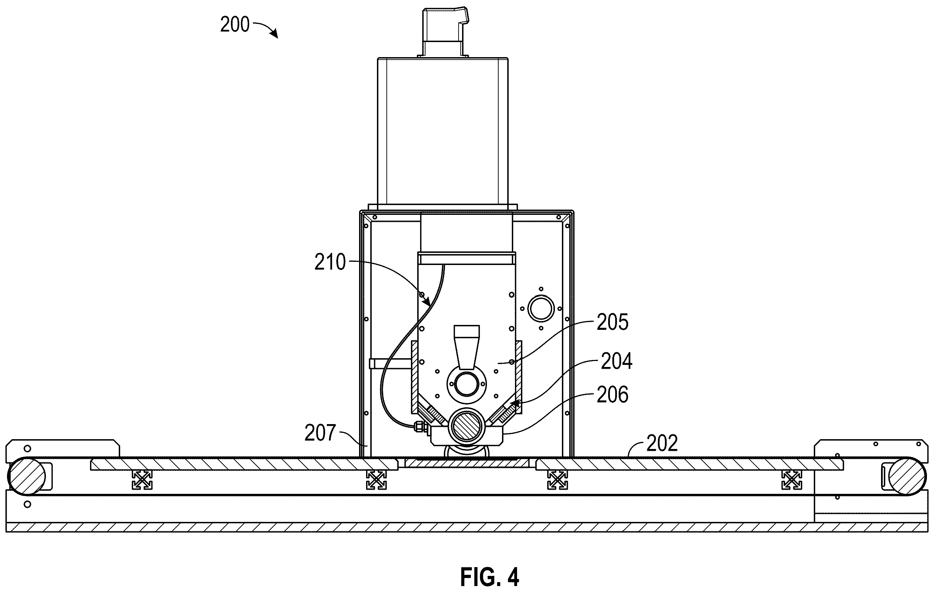

Fig. 4 shows a side view diagram of an apparatus 200 as described herein for treating fine particles to attach infectious bacteriophage thereto, as is also described herein

### Example 1

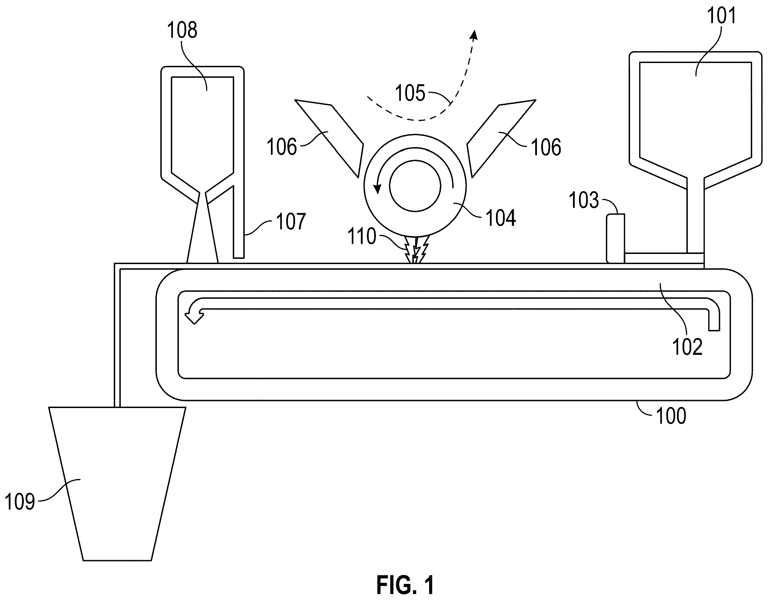

An apparatus 100 for treating fine particles to attach infectious bacteriophage thereto is shown in Fig. 1.

The following components of the apparatus are shown in Fig. 1:
101 - Hopper containing fine particles, which are deposited on the belt;
102 - Moving belt which is made of conducting material, or is made to have conducting properties by the addition of a second electrode. This grounds the particles, thus greatly reducing or eliminating the issues with static build up;
103 - Barrier across belt ensures that a fine (monolayer) of fine particles enter the corona area;
104 - Rotating ceramic electrode which generates a corona to activate the material. The rotating electrode allows cooling of the top surface via channelled air flow;
105 - Air is channelled over the top of the electrode to allow cooling;
106 - Silicon polymer blades isolate feed from the air flow and scrape the electrode surface of any particles which attach;
107 - Barrier to prevent liquid coming into contact with the corona;
108 - Device to spray phage suspension onto activated feed with reservoir for same;
109 - Hopper to collect phage-immobilised feed.

Thus in operation the moving (conveyor) belt 102 has fine particles deposited on it from a hopper 101. The particles are of less than or equal to 2 mm such that static electric effects might cause them to be moved at spread or dispersed in an uncontrolled manner. The particles deposited on the belt travel beneath a barrier 103 running transversely across the belt 102 in order to shape and flatten the mass of particles on the belt 102 and into a monolayer in preparation for bulk treatment of the particles.

The conveyor surface is made of a conducting material or its electrical potential is manipulated by way of using a second electrode. This serves to earth or ground the particles in order to eliminate static electricity and thus the movement of fine particles on the conveyor due to static electricity. Such effects are particularly pronounced when the particles are formed into a monolayer.

The monolayer of particles travelling on the conveyor then pass through a corona discharge 110 which causes activation of the surface of the particles. Activation is the generation of reactive free radicals on the surface of the particles.

The electrode that produces the corona discharge 104 is preferably ceramic, because the conveyor belt is conducting and thus a ceramic electrode is preferred for compatibility. In addition, the ceramic electrode 104 is cylindrical and rotates while producing the corona discharge 110. The lowest part of the curved surface of the cylindrical electrode 104 is arranged transversely to the direction of movement of the conveyor belt and faces the particles travelling on the conveyor belt 102. The electrode produces the corona discharge from this lowest portion of its surface that faces the conveyor belt.

Concomitant rotation of the cylindrical electrode 104 while producing the corona discharge means that the active portion of the electrode is constantly changing as a previously inactive portion of the electrode is brought into use as the electrode surface rotates. Production of corona discharge produces heat and causes the temperature of the active portion of the electrode to rise. Rotation of the electrode means that the previously active portion of the electrode 104 can be rotated into a cooling area 105 wherein air is channelled over the inactive, upper, portion of the electrode 104 in order to cool it before that section of the electrode becomes active once more.

In addition, silicone polymer blades 106 are arranged in close and parallel separation along the length of the curved cylindrical surface of the ceramic electrode. The presence of these silicone polymer blades 106 allows the separation of the atmosphere on the "active" side of the electrode from the atmosphere on the "inactive" side of the electrode. In this way the effectiveness of the cooling system by which air is passed over the "inactive" side of the electrode is improved and the passage of cooling air on the "inactive" side is prevented from blowing on or affecting affect the fragile monolayer of fine particles born on the conveyor belt facing the "active" side of the electrode. In addition, the arrangement of the blades 106 to be in close and parallel separation with these curved surface of the electrode 104 means that particles that may have adhered to the surface of the electrode because of a static charge or for other reasons are scraped off the electrode it rotates during the treatment process.

The activated particles on the conveyor then pass through an aperture in a screen 107 into a spraying area wherein a suspension of bacteriophage in a carrier liquid is sprayed from a reservoir 108 onto the activated particles. Spraying the bacteriophage onto the activated particles means that the surface of the bacteriophage reacts with the free radicals present on the surface of the activated particles in order to covalently link the bacteriophage to the particles. The bacteriophage attached in this way can retain infectivity and are significantly less susceptible to dehydration, chemical activation or predation. The suspension contains 1 × 10⁴ between and 1 x 10¹² bacteriophage per ml. The suspension is applied by way of passing the suspension through a spray nozzle to form a spray formed of droplets with an average diameter of Y µm. A pump is used to apply the suspension at a flow rate of Y ml per second. The spray nozzle is located Y mm from the activated phage whilst the bacteriophage-containing spray is applied.

In once the bacteriophage have been covalently attached to particles the conveyor takes the particles to a storage hopper 109

### Example 2

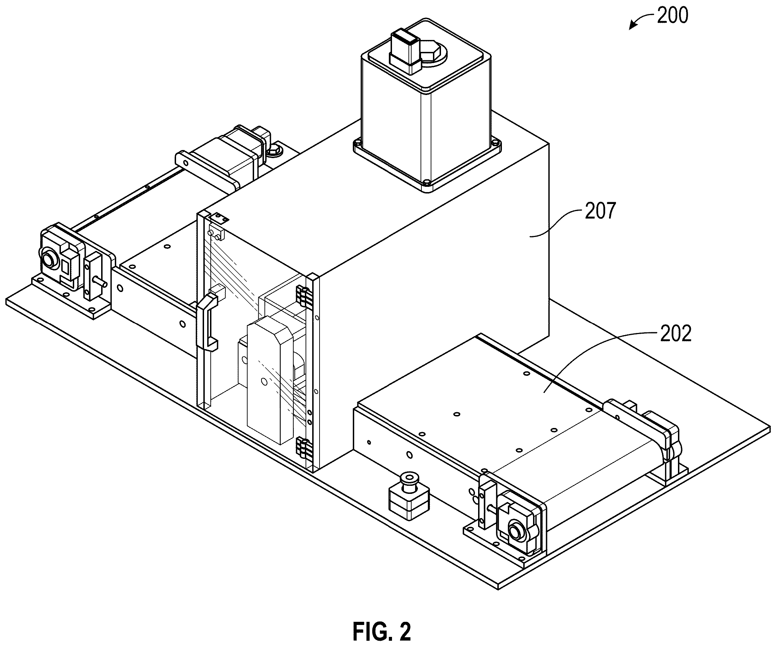

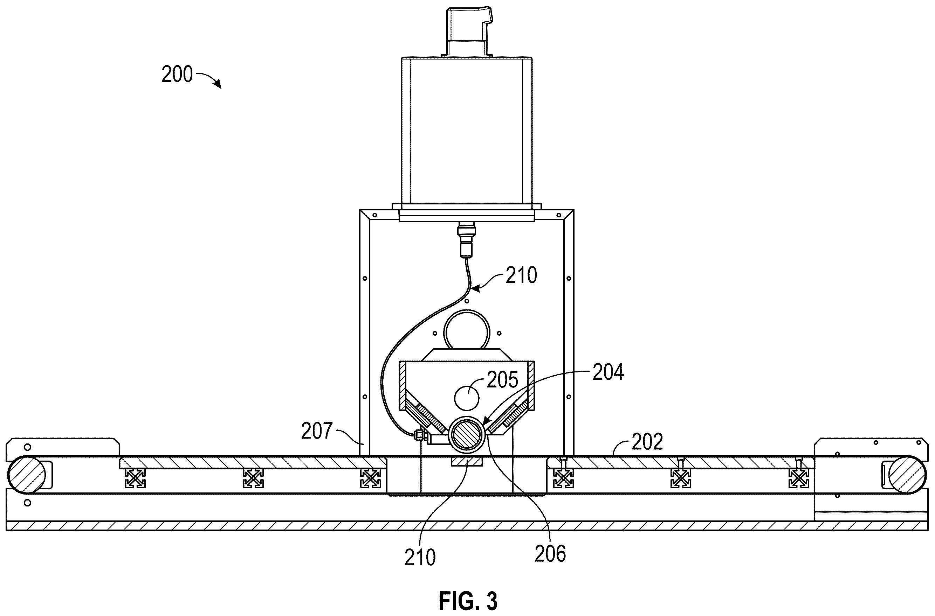

An apparatus 200 for treating fine particles to attach infectious bacteriophage thereto is shown in Figs 2 and 3.

The following components of the apparatus are shown in Figs. 2 and 3:
202 - Moving belt whose electrical potential and the electrical potential of items borne thereon is controlled via the second/counter electrode 210. This grounds the particles, thus greatly reducing or eliminating the issues with static build up;
204 - Rotating ceramic electrode which generates a corona to activate the material. The rotating electrode allows cooling of the top surface via channelled air flow;
205 - Cooling pipe that supplies/channels air over the top of the electrode to allow cooling;
206 - Silicon polymer blade to isolate feed from the air flow and scrape the electrode surface of any particles which attach thereto;
207 - Barrier to prevent liquid coming into contact with the corona;
210 - Counter electrode; and
212 - High voltage (HV) cable.

Thus in operation the moving (conveyor) belt 202 has fine particles deposited on it from a hopper or other container (not shown). The particles are of a size such that static electric effects might cause them to be moved at spread or dispersed in an uncontrolled manner. The particles deposited on the belt travel beneath a barrier (not shown) running transversely across the belt 202 in order to shape and flatten the mass of particles on the belt 202 and into a monolayer in preparation for bulk treatment of the particles.

The electrical potential of the conveyor surface is manipulated by way of using a second electrode that acts as a counter electrode. This serves to control static electric charges borne by the particles and thus control or prevent the movement of fine particles on the conveyor due to static electricity. Such effects are particularly pronounced when the particles are formed into a monolayer.

The monolayer of particles travelling on the conveyor then pass through a corona discharge which causes activation of the surface of the particles. Activation is the generation of reactive free radicals on the surface of the particles.

The electrode that produces the corona discharge 204 is ceramic. In addition, the ceramic electrode 204 is cylindrical and rotates while producing the corona discharge. The lowest part of the curved surface of the cylindrical electrode 204 is arranged transversely to the direction of movement of the conveyor belt and faces the particles travelling on the conveyor belt 202. The electrode produces the corona discharge from this lowest portion of its surface that faces the conveyor belt. The electrical energy for generating the corona discharge is supplied via a high-voltage (HV) cable 212. The HV cable is held a safety distance of 60 mm from other conductive material.

Concomitant rotation of the cylindrical electrode 204 while producing the corona discharge means that the active portion of the electrode is constantly changing as a previously inactive portion of the electrode is brought into use as the electrode surface rotates. Production of corona discharge produces heat and causes the temperature of the active portion of the electrode to rise. Rotation of the electrode means that the previously active portion of the electrode 204 can be rotated into the vicinity of a cooling pipe 205 wherein air is channelled over the inactive, upper, portion of the electrode 204 in order to cool it before that section of the electrode becomes active once more.

In addition, silicone polymer blades 206 are arranged in close and parallel separation along the length of the curved cylindrical surface of the ceramic electrode. The presence of these silicone polymer blades 206 allows the separation of the atmosphere on the "active" side of the electrode from the atmosphere on the "inactive" side of the electrode. In this way the effectiveness of the cooling system by which air is passed over the "inactive" side of the electrode is improved and the passage of cooling air on the "inactive" side is prevented from blowing on or affecting affect the fragile monolayer of fine particles born on the conveyor belt facing the "active" side of the electrode. In addition, the arrangement of the blades 206 to be in close and parallel separation with these curved surface of the electrode 204 means that particles that may have adhered to the surface of the electrode because of a static charge or for other reasons are scraped off the electrode it rotates during the treatment process.

The activated particles on the conveyor then pass through an aperture in a screen (not shown) into a spraying area wherein a suspension of bacteriophage in a carrier liquid is sprayed from a reservoir (not shown) onto the activated particles. Spraying the bacteriophage onto the activated particles means that the surface of the bacteriophage reacts with the free radicals present on the surface of the activated particles in order to covalently link the bacteriophage to the particles. The bacteriophage attached in this way can retain infectivity and are significantly less susceptible to dehydration, chemical activation or predation. The suspension contains 1 × 10⁴ to 1 x 10¹² bacteriophage per ml. The suspension is applied by way of passing the suspension through a spray nozzle to form a spray formed of droplets.

In once the bacteriophage have been covalently attached to particles the conveyor takes the particles to a storage hopper (not shown)

The invention thus provides methods and apparatus for treating substrates in order to attach substances thereto, uses of the apparatus and products provided by applying a method of the invention using the apparatus.

### References

[1] C. Wang, D. Sauvageau, A. Elias Immobilization of Active Bacteriophages on Polyhydroxyalkanoate Surfaces. ACS Appl. Mater. Interfaces 2016, 8, 1128-1138
[2] Barton, D.; Short, R. D.; Fraser, S.; Bradley, J. W. The Effect of Ion Energy upon Plasma Polymerization Deposition Rate for Acrylic Acid. Chem. Commun. (Cambridge, U. K) 2003, 7, 348-349.
[3] Canavan, H. E.; Cheng, X.; Graham, D. J.; Ratner, B. D.; Castner, D. G. A Plasma-Deposited Surface for Cell Sheet Engineerning: Advantages over Mechanical Dissociation of Cells. Plasma Processes Polym. 2006, 3, 516-523.
[4] Recek, N.; Mozetic, M.; Jaganjac, M.; Milkovic, L.; Zarkovic, N.; Vesel, A. Adsorption of Proteins and Cell Adhesion to Plasma Treated Polymer Substrates. Int. J. Polym. Mater. 2014, 63, 685-691.
[5] Kerkeni, A.; Behary, N.; Dhulster, P.; Chihib, N.; Perwuelz, A. Study on the Effect of Plasma Treatment of Woven Polyester Fabrics with Respect to Nisin Adsorption and Antibacterial Activity. J. Appl. Polym. Sci. 2013, 129, 866-873.

## Claims

1. An electrode assembly for exposing an object supported on a moving surface to an electrical discharge comprising:
a) an electrical discharge device capable of generating an electrical discharge, further comprising an electrode having an electrical discharge generating surface capable of generating an electrical discharge originating from an active portion of the surface, wherein the surface is movable in order to allow generation of an electrical discharge from a different portion of the surface;
b) a barrier located in closely spaced separation to an inactive area of the electrode wherein the barrier separates the active portion of the electrode from an inactive portion of the electrode; and
c) means for cooling the inactive portion of the electrode.

2. An electrode assembly according to claim 1, wherein the electrical discharge is generated continuously while the electrical discharge generating surface moves.

3. An electrode assembly according to claim 1 or claim 2, wherein the electrical discharge is a corona discharge.

4. An electrode assembly according to any of claims 1 to 3, wherein the means for cooling the inactive portion of the electrode is air cooling, preferably forced air cooling.

5. An electrode assembly according to any of claims 1 to 4, wherein the barrier is made of rubber or plastic.

6. An electrode assembly according to claim 6, wherein the barrier is made of silicone material.

7. An electrode assembly according to any of claims 1 to 6, wherein the corona discharge generating electrode is ceramic.

8. An electrode assembly according to any of claims 1 to 6, wherein the corona discharge generating electrode is metal.

9. A method of exposing an object to an electrical discharge comprising the step of exposing the object to an electrical discharge generated by the electrode assembly of any of claims 1 to 8.

10. A method according to claim 9 for of attaching a molecule or macromolecule to an object comprising the steps of:
a) activating the object by exposing it to an electrical discharge generated by the electrode assembly of any of claims 1 to 8; and
b) contacting the object with the molecule or macromolecule to be attached.

11. A method of claim 10 for attaching a molecule or macromolecule to an object, wherein the molecule or macromolecule is a bacteriophage.

12. An apparatus for exposing an object to an electrical discharge comprising the electrode assembly of any of claims 1 to 8.

13. Use of the electrode assembly of any of claims 1 to 8 for exposing an object to an electrical discharge.
